# EUROPEAN PATENT SPECIFICATION

(11) **EP 1 883 443 B1**
(45) Date of publication and mention of the grant of the patent: **03.03.2010**
(21) Application number: 06771252.1
(22) Date of filing: 26.05.2006
(51) Int. Cl.: A61M 25/09, A61M 25/06

(54) **Low profile introducer apparatus**
Niedrigprofil-Einführvorrichtung
Appareil introducteur peu encombrant

(30) Priority: 27.05.2005 US 685188 P
(43) Date of publication of application: 06.02.2008
(73) Proprietor: Cook Incorporated, Bloomington, Indiana 47402-0489 (US)
(72) Inventor: VALAIE, Arman, H., Bloomington, IN 47403 (US)
(74) Representative: Mathys & Squire LLP
(86) International application number: PCT/US2006/020368
(87) International publication number: WO 2006/130451

(56) References cited:
- EP-A- 0 778 045
- EP-A- 1 092 449
- WO-A-93/02735
- WO-A-2005/004967
- US-A- 4 404 159
- US-A- 4 405 314
- US-A- 4 629 450
- US-A- 4 650 472
- US-A- 6 053 904

## Description

### Technical Field

This invention relates to the field of percutaneous access to the vascular system. More specifically, this invention relates to a low profile apparatus used to introduce catheters and other interventional devices into the vascular system with small gauge needles.

### Background of the Invention

Many medical procedures require the percutaneous placement of an interventional medical device, such as a catheter, into an artery or vein. Such interventional devices are used for, among otherthings, blood pressure monitoring, blood sampling, and the administration of fluids and medicaments to a patient.

Typically, such devices are introduced using the well-known Seldinger percutaneous entry technique. The Seldinger technique for percutaneous entry into the vascular system has been in widespread use in diagnostic and interventional medicine for many years. In the Seldinger technique, the physician makes an oblique entry into the artery or vein with a beveled needle. A wire guide is passed into the proximal end of the needle, through the length of the needle and into the artery or vein. The needle is thereafter withdrawn, leaving the wire guide in place. The catheter or other interventional device is then passed over the wire guide, through the puncture, and into the artery or vein at the needle puncture site. Once the catheter is in place, the wire guide can be withdrawn.

One of the disadvantages of this procedure is that the initial needle stick must be made with a needle that is large enough to accept the wire guide through its central bore. Conventional wire guides are normally comprised of a tightly wound helical stainless steel wire coil. In order to have sufficient rigidity to properly support and lead many standard catheters and other interventional devices in common use in modem medicine, such wire guides are typically constructed to have an outer diameter (O. D.) in the range of about 0.89 or 0.97 mm (0.035 to 0.038 inch). This diameter of wire guide will typically pass through an 18 gauge thinwall needle. An 18 gauge needle typically has a 1.27 mm (0.050 inch) outer diameter and a 1.07 mm (0.042 inch) inner diameter.

The 18 gauge needle is the most common sized needle used for initial vascular access, and has become a standard needle for use with the Seldinger technique for percutaneous catheterization. However, the outer diameter of an 18 gauge needle is just large enough to damage tissue or cause excessive bleeding if it does not enter the vessel correctly, or if it inadvertently penetrates an organ or other unintended body structure. As a result, it is desirable to utilize a smaller gauge needle to effect the initial entry. Needles of 21 gauge thinwall, or smaller, are considered small enough that they do not damage tissue or organs, or cause excessive bleeding if inserted off target. In addition, needles having smaller outer diameters generally have correspondingly shorter bevels at the needle tip compared to the size of the bevel tip of an 18 gauge needle. Thus, it is much easier to get a short bevel into the lumen of a small vessel than the longer bevel of the 18 gauge needle.

Unfortunately, the bore of a needle of 21 gauge, or smaller, is not large enough to pass a standard 0.89 mm or 0.97 mm (0.035 inch or 0.038 inch) diameter wire guide therethrough. The largest wire guide that can be easily introduced into such small gauge needles is normally a wire of 0.46 mm (0.018 inch) outer diameter. However, many diagnostic and interventional devices need at least a 0.89 mm (0.035 inch), and more preferably a 0.97 mm (0.038 inch), diameter wire guide to provide sufficient support to optimally introduce and manipulate such devices through the vasculature. Thus, unless and until a larger diameter wire guide is introduced into the vasculature, many such devices cannot be introduced. U.S. Patent No.4,650,472 ("the '472 patent"), assigned to the assignee herein, describes a catheterization apparatus which allows a smaller gauge needle, such as a 22 gauge (0.72 mm; 0.028 inch O.D.) needle, to be used for the initial puncture through the skin of the patient in place of the larger conventional 18 gauge needle.

A 0.46 mm (0.018 inch) outer diameter wire guide is initially inserted through the bore of the small gauge (e.g. 22 gauge) needle. The needle is thereafter withdrawn, and a removable inner cannula, or dilator, is provided over the wire guide but inside an outer sleeve portion of the catheterization apparatus. This removable inner cannula has a tapered tip, and provides a transition between the large distal opening of the outer sleeve and the 0.46 mm (0.018 inch) wire guide. The inner cannula is generally about 0.97 mm (0.038 inch) O.D., and the outer sleeve is sized to fit over the inner cannula.

The outer sleeve and the inner cannula of the apparatus disclosed in the '472 patent are normally inserted into the blood vessel in tandem. The diametrical transition of the leading end of this tandem is intended to minimize the trauma that may otherwise be caused by the insertion of a large diameter outer sleeve over a small diameter wire guide. Once the outer sleeve is properly positioned within the blood vessel, the inner cannula and the smaller wire guide can be withdrawn, leaving the outer sleeve in place. A larger 0.89 to 0.97 mm (0.035 to 0.038 inch) wire guide can then be introduced through the outer sleeve and into the vessel. The outer sleeve can thereafter be removed from the patient, leaving the larger wire guide in the vessel ready to accept a catheter or other interventional device, as in the standard Seldinger technique. The apparatus of the '472 patent has been successfully used to percutaneously insert a catheter having a large diameter O.D. into a blood vessel when the initial insertion is made with an introducer needle and a wire guide which are much smaller in diameter than the distal opening of the catheter.

The apparatus of the '472 patent thus enables the physician to introduce larger diagnostic and interventional devices into a vessel than would otherwise be possible when the initial vessel entry is made with a small gauge needle. When the apparatus is inserted into the vessel as described, however, the physician must exert sufficient force to overcome the resistance provided at the "bump" that is present at the transition between the distal end of the outer sleeve and the underlying portion of the inner cannula. In addition, if the amount of force is not carefully controlled, the side of the vessel opposite the initial stick may be punctured. Although such insertions may be safely performed, it is nonetheless desired to further minimize the amount of force required to insert the outer sleeve into the vessel, and to reduce the possibility that the physician may inadvertently puncture any portion of the vessel wall.

### Brief Description of the Drawing

Fig. 1 is a side view of a prior art apparatus for effecting catheterization of a body vessel using a small gauge introducer needle;
Fig. 2 is a sectional view of the outer sleeve of the apparatus of Fig. 1;
Fig. 3 illustrates one embodiment of a wire guide exchange apparatus of the present invention;
Fig. 4 is a sectional view of the outer sleeve of the apparatus of Fig. 3;
Fig. 5 is a graph illustrating the relationship between compressive extension and compressive load for specimens 1-15 of an embodiment of the inventive apparatus;
Fig. 6 is a graph illustrating the relationship between compressive extension and compressive load for specimens 16-30 of an embodiment of the inventive apparatus;
Fig. 7 is a graph illustrating of relationship between compressive extension and compressive load for specimens 1-15 of a reference apparatus; and
Fig. 8 is a graph illustrating the relationship between compressive extension and compressive load for specimens 16-30 of a reference apparatus

### Detailed Description

For the purposes of promoting an understanding of the principles of the invention, reference will now be made to the embodiments illustrated in the drawings, and specific language will be used to describe the same. It should nevertheless be understood that no limitation of the scope of the invention is thereby intended, such alterations and further modifications in the illustrated device, and such further applications of the principles of the invention as illustrated therein being contemplated as would normally occur to one skilled in the art to which the invention relates.

In the following discussion, the terms "proximal" and "distal" will be used to describe the opposing axial ends of the inventive apparatus, as well as the axial ends of various related components. The term "proximal" is used in its conventional sense to refer to the end of the apparatus (or component) that is closest to the operator during use of the collar. The term "distal" is used in its conventional sense to refer to the end of the apparatus (or component) that is initially inserted into the patient, or that is closest to the patient.

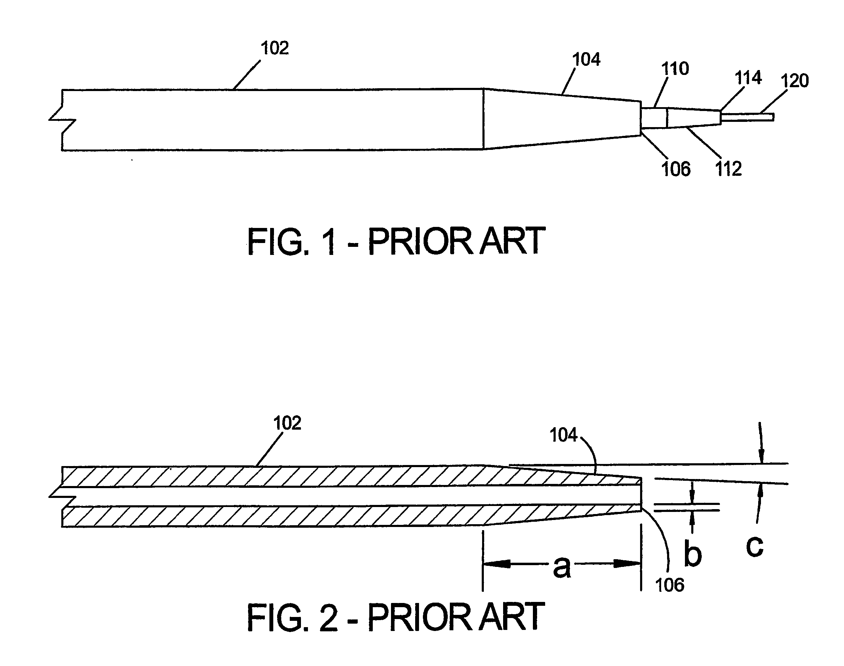

Fig. 1 illustrates one embodiment of a prior art apparatus 100 for effecting catheterization of a body vessel using a small gauge introducer needle. The apparatus shown in Fig. 1 is further described in U.S. Patent No. 4,650,472. Fig. 1 illustrates the dimensional relationship between outer sleeve 102, inner cannula 110 and wire guide 120 at the distal end portion of prior art apparatus 100. Outer sleeve 102 includes a distal portion 104 that tapers to outer sleeve distal end 106. Inner cannula 110 also includes a distal portion 112 that tapers to inner cannula distal end 114. Wire guide 120 extends from inner cannula distal end 114 in the normal fashion.

Fig. 2 is a sectional view of outer sleeve 102, shown removed from apparatus 100. Generally, tapered area a in this prior art apparatus is approximately 4 (±1) mm long. The thickness b of the wall of sleeve 102 at distal end 106 is about 0.1016 (±0.0005) mm (0.004 inch).

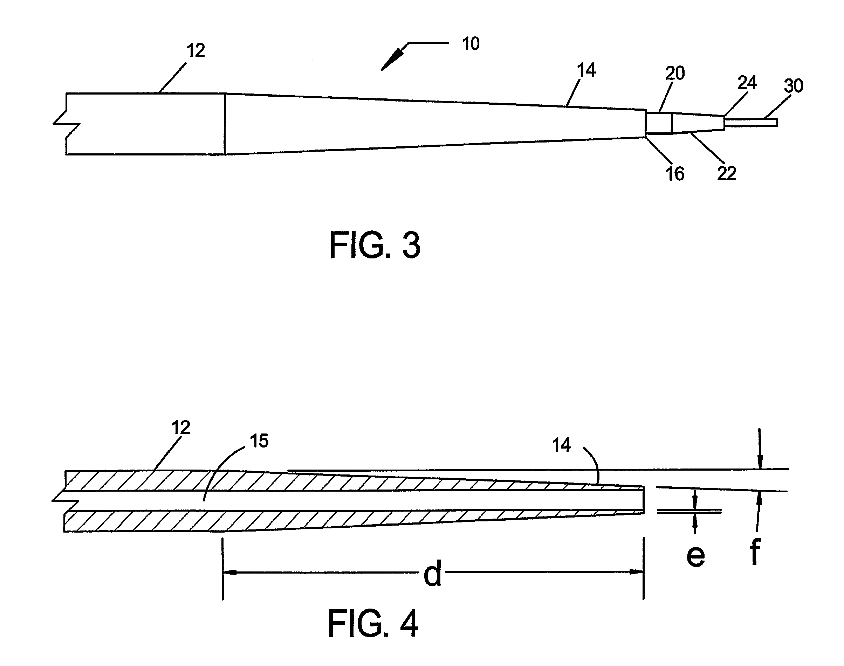

Fig. 4 is a sectional view of the outer sleeve of the apparatus of Fig. 3;

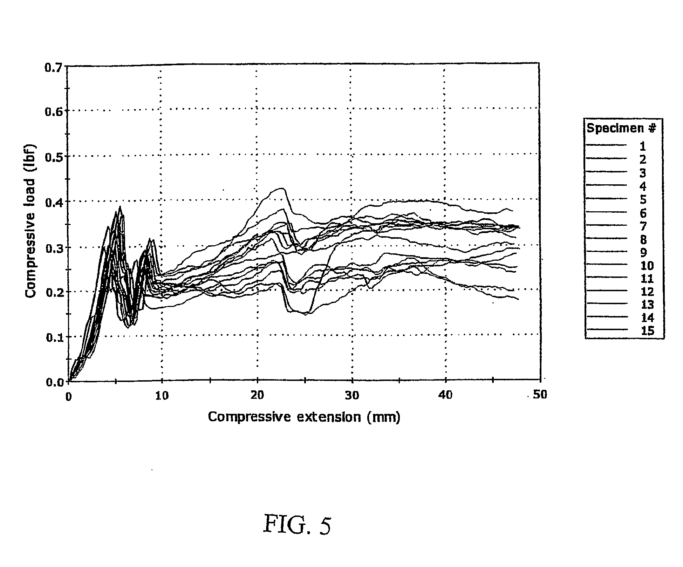

Fig. 5 is a graph illustrating the relationship between compressive extension and compressive load for specimens 1-15 of an embodiment of the inventive apparatus;

The thickness e of the wall of sleeve 12 at distal end 16 is less than 0.076 mm (0.003 inch). Preferably, thickness e is between about 0.0127 mm (0.0005) and 0.038 mm (0.0015 inch), and most preferably, about 0.0254 mm (0.001 inch). The angle f of taper of tapered area 14 from the longitudinal axis is less than about 2.5°, preferably between about 0.5° and about 2°, and more preferably between 0.5° and 2°, or from about 1 ° to about 1.5° and most preferably 1°. The exact angle of taper will preferably correspond to the remaining dimensions of the outer sleeve and inner cannula such that a generally smooth transition is provided between the distal end of the sleeve and the outer surface of the inner cannula.

The inventive apparatus 10 can be of any conventional size for its intended purposes. Preferably, however, the outer sleeve 12 is between about 1.32 mm and 1.98 mm (4 and 6 French) and most preferably, about 1.65 mm (5 French). The lumen 15 of the outer sleeve preferably has a diameter of about 1.016 mm (0.04 inch). Inner cannula 20 is sized to fit within the lumen of outer sleeve 12 in conventional fashion for such devices. Those skilled in the art will appreciate that the dimensions for a particular sheath to be used for a particular purpose, and that sheaths of other dimensions may be similarly made within the scope of the invention.

One example of the gradual transition of the outer surface of outer sleeve 12 of the inventive apparatus to the inner cannula 20 is shown in Fig. 3. This transition is much smoother, avoids the substantial shoulder represented by the dimension b of Figure 2, and occurs in a much more gradual manner over a greater tapered length 14 of the outer sleeve, when compared to the much more abrupt transition of the tapered portion 104 of the prior art device shown in Fig. 1. The smooth, gradual transition of the inventive apparatus provides a very sleek profile that enables the apparatus to be smoothly inserted into, and passed through, the initial body opening and the underlying tissue. This results in a reduction of the trauma experienced by the patient at the insertion point.

The step or shoulder represented by the wall thickness of the outer sleeve at the distal end is preferably 10% or less than the external radius of the outer sleeve at its full (non-tapered) diameter, more preferably 5% or less, still more preferably 3% or less.

To further illustrate this reduction in trauma, tests were performed to simulate the force required to be exerted by a physician during the insertion of an inventive introducer apparatus through the skin at a body opening. For comparison, similar tests were performed utilizing a conventional introducer apparatus. A sheet of 0.965 mm (0.038 inch) duro silicone with a translucent color (available from AAA-Acme Rubber Company of Tempe, Arizona), was provided to simulate the skin of a patient. In each case, the initial puncture through the silicone sheet was made with a conventional 21 gauge needle. A 0.46 mm (0.018 inch) wire guide was inserted through the bore of the needle in conventional fashion. The needle was thereafter withdrawn over the wire guide, and the sheath/cannula apparatus was inserted over the wire guide. Each apparatus included a 1.65 mm (5 French) outer sleeve, and a 0.99 mm (3 French) inner cannula (dilator).

The inventive apparatus included an outer sleeve having a tapered portion of about 15 mm, and forming an angle of about 1 with the longitudinal axis. The thickness of the wall of the outer sleeve at its distal end was about 0.0254 mm (0.001 inch). The comparative reference apparatus included an outer sleeve having a tapered portion of about 4 mm, and forming an angle of about 3° with the longitudinal axis. The thickness of the wall of the outer sleeve at its distal end was about 0.1016 mm (0.004 inch).

Simulations were performed on thirty specimens of the inventive apparatus and thirty specimens of the conventional apparatus. The testing conditions were identical on all specimens, with the exception of the structural differences in the outer sleeves of the respective apparatuses as described. The tests were designed to simulate the compressive load (in newton) that is exerted on the skin as the introducer apparatus initially enters the skin through the puncture, and as the introducer apparatus is continuously inserted to a depth (or compressive extension) of about 50 mm.

The results of the tests on the 30 specimens of the inventive apparatus are shown below in Table 1.

**TABLE 1:**

| Specimen Label | Maximum load Newton (lbf) | Compressive extension at Maximum Comp. load (mm) | Compressive load at 47 mm Newton (lbf) | Comment |
|---|---|---|---|---|
| 1 | 1.38(0.31) | 4.47458 | 1.24 (0.27933) | Pushed without incident |
| 2 | 1.56 (0.35) | 4.18979 | 1.46 (0.32828) | Pushed without incident |
| 3 | 1.42 (0.32) | 32.73474 | 1.28 (0.28809) | Pushed without incident |
| 4 | 1.78 (0.40) | 36.24515 | 1.19 (0.26727) | Pushed without incident |
| 5 | 1.73 (0.39) | 5.53499 | 1.07 (0.24153) | Pushed without incident |
| 6 | 1.47 (0.33) | 5.00999 | 1.05 (0.23552) | Pushed without incident |
| 7 | 1.60 (0.36) | 35.05036 | 0.91 (0.20435) | Pushed without incident |
| 8 | 1.29 (0.29) | 4.70999 | 1.06 (0.23899) | Pushed without incident |
| 9 | 1.25 (0.28) | 47.49513 | 1.10 (0.24798) | Pushed without incident |
| 10 | 1.60 (0.36) | 5.23041 | 1.02 (0.23015) | Pushed without incident |
| 11 | 1.56 (0.35) | 41.46015 | 0.84 (0.18996) | Pushed without incident |
| 12 | 1.91 (0.43) | 22.76017 | 1.15 (0.25893) | Pushed without incident |
| 13 | 1.65 (0.37) | 5.86520 | 0.82 (0.18430) | Pushed without incident |
| 14 | 1.51 (0.34) | 4.84437 | 1.16 (0.26116) | Pushed without incident |
| 15 | 1.29 (0.29) | 46.74514 | 0.99 (0.22182) | Pushed without incident |
| 16 | 1.47 (0.33) | 34.20495 | 1.08 (0.24218) | Pushed without incident |
| 17 | 1.56 (0.35) | 5.20999 | 1.13 (0.25428) | Pushed without incident |
| 18 | 1.60 (0.36) | 5.63499 | 0.95 (0.21307) | Pushed without incident |
| 19 | 1.60 (0.36) | 5.34978 | 0.95 (0.21285) | Pushed without incident |
| 20 | 1.65 (0.37) | 47.17951 | 0.83 (0.18557) | Pushed without incident |
| 21 | 1.60 (0.36) | 46.88972 | 1.07 (0.24057) | Pushed without incident |
| 22 | 1.60 (0.36) | 5.47499 | 1.01 (0.22677) | Pushed without incident |
| 23 | 1.69 (0.38) | 6.07978 | 0.86 (0.19394) | Pushed without incident |
| 24 | 1.47 (0.33) | 5.61041 | 0.81 (0.18142) | Pushed without incident |
| 25 | 1.25 (0.28) | 4.99520 | 0.87 (0.19651) | Pushed without incident |
| 26 | 1.47 (0.33) | 41.46952 | 0.87 (0.19551) | Pushed without incident |
| 27 | 1.73 (0:39) | 5.98520 | 0.73 (0.16382) | Pushed without incident |
| 28 | 1.65 (0.37) | 36.46015 | 0.70 (0.15755) | Pushed without incident |
| 29 | 1.69 (0.38) | 5.68999 | 0.92 (0.20767) | Pushed without incident |
| 30 | 1.51 (0.34) | 40.59056 | 0.76 (0.17002) | Pushed without incident |
| Mean | 1.56 (0.35) | 19.97250 | 0.10 (0.2398) | |
| Standard Deviation | 0.15 (0.03480) | 17.70996 | 0.16 (0.03969) | |
| Minimum | 1.25 (0.28) | 4.18979 | 0.70 (0.15755) | |
| Maximum | 1.91 (0.43) | 47.49513 | 1.46 (0.32828) | |

| | | | | |
|---|---|---|---|---|
| Rate 115.0 mm/min Data captureManual Control mode 1Compressive extension Start of test Temperature (deg C)21.0 Start of Test Relative Humidity (%)37.0 End of test Temperature (deg C)21.0 End of test Relative Humidity (%)29.0 | | | | |

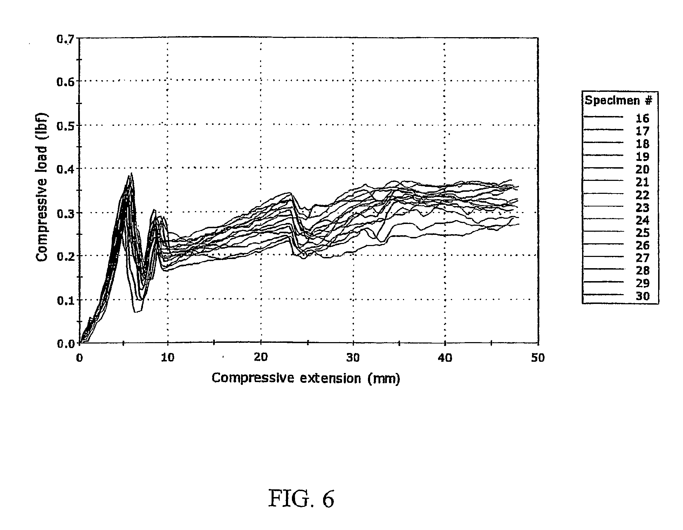

The data of Table 1 is illustrated graphically in Fig. 5 for specimens 1 to 15, and in Fig. 6 for specimens 16 to 30 of the inventive apparatus. In the figures, the "zero point" ("0") of the "compressive extension" and the "compressive load" represents the point where the inner cannula initially touches the sheet as it is urged forwardly for insertion.

The results of the tests on the 30 specimens of the conventional, reference apparatus are shown below in Table 2.

**TABLE 2:**

| Specimen Label | Maximum load Newton (lbf) | Compressive extension at Maximum Comp. load (mm) | Compressive load at 47 mm Newton (lbf) | Comment |
|---|---|---|---|---|
| 1 | 2.67 (0.60) | 11.28040 | 0.77 (0.17221) | Pushed without incident |
| 2 | 2.76 (0.62) | 11.46957 | 0.60 (0.13470) | Pushed without incident |
| 3 | 2.71 (0.61) | 9.84978 | 0.96 (0.21689) | Pushed without incident |
| 4 | 2.71(0.61) | 10.67436 | 0.80 (0.17909) | Pushed without incident |
| 5 | 2.54 (0.57) | 9.78998 | 1.11 (0.24985) | Pushed without incident |
| 6 | 2.71 (0.61) | 9.47436 | 1.25 (0.28167) | Pushed without incident |
| 7 | 2.62 (0.59) | 10.46415 | 0.96 (0.21618) | Pushed without incident |
| 8 | 2.62 (0.59) | 11.01998 | 0.64 (0.14473) | Pushed without incident |
| 9 | 2.54 (0.57) | 9.48040 | 1.27 (0.28587) | Pushed without incident |
| 10 | 2.80 (0.63) | 10.59957 | 0.88 (0.19785) | Pushed without incident |
| 11 | 2.67 (0.60) | 10.93436 | 0.71 (0.15891) | Pushed without incident |
| 12 | 2.76 (0.62) | 10.51498 | 0.82 (0.18479) | Pushed without incident |
| 13 | 2.62 (0.59) | 10.62915 | 0.81 (0.18130) | Pushed without incident |
| 14 | 2.71 (0.61) | 11.20019 | 0.74 (0.16731) | Pushed without incident |
| 15 | 2.54 (0.57) | 10.39457 | 0.83 (0.18708) | Pushed without incident |
| 16 | 2.98 (0.67) | 10.64936 | 1.01 (0.22800) | Pushed without incident |
| 17 | 2.58 (0.58) | 9.64540 | 1.02 (0.28844) | Pushed without incident |
| 18 | 2.54 (0.57) | 10.12457 | 0.93 (0.21000) | Pushed without incident |
| 19 | 2.54 (0.57) | 9.97436 | 0.97 (0.21853) | Pushed without incident |
| 20 | 2.80 (0.63) | 10.49519 | 0.92 (0.20705) | Pushed without incident |
| 21 | 2.62 (0.59) | 10.71936 | 0.79 (0.17773) | Pushed without incident |
| 22 | 2.71 (0.61) | 11.48415 | 0.55 (0.12394) | Pushed without incident |
| 23 | 2.67 (0.60) | 9.99478 | 1.05 (0.23544) | Pushed without incident |
| 24 | 2.67 (0.60) | 10.29978 | 1.01 (0.22631) | Pushed without incident |
| 25 | 2.54 (0.57) | 10.59540 | 0.97 (0.21824) | Pushed without incident |
| 26 | 2.54 (0.57) | 10.41957 | 0.84 (0.18823) | Pushed without incident |
| 27 | 2.54 (0.57) | 11.18477 | 0.44 (0.09785) | Pushed without incident |
| 28 | 2.36 (0.53) | 9.67999 | 1.20 (0.26979) | Pushed without incident |
| 29 | 2.58 (0.58) | 10.18103 | 1.17 (0.26243) | Pushed without incident |
| 30 | 2.76 (0.62) | 10.91498 | 0.72 (0.16288) | Pushed without incident |
| Mean | 2.67 (0.60) | 10.47128 | 0.90 (0.20244) | |
| Standard Deviation | 0.12 (0.02679) | 0.57158 | 0.21 (0.04818) | |
| Minimum | 2.36 (0.53) | 9.47436 | 0.44 (0.09785) | |
| Maximum | 2.98 (0.67) | 11.48415 | 1.02 (0.28844) | |

| | | | | |
|---|---|---|---|---|
| Rate 115.0 mm/min Data captureManual Control mode 1 Compressive extension Start of test Temperature (deg C)21.0 Start of Test Relative Humidity (%)32.0 End of test Temperature (deg C)21.0 End of test Relative Humidity (%)30.0 | | | | |

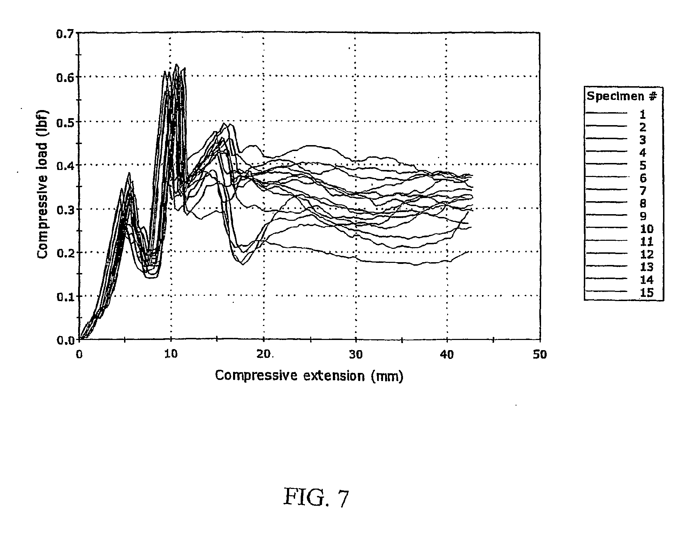

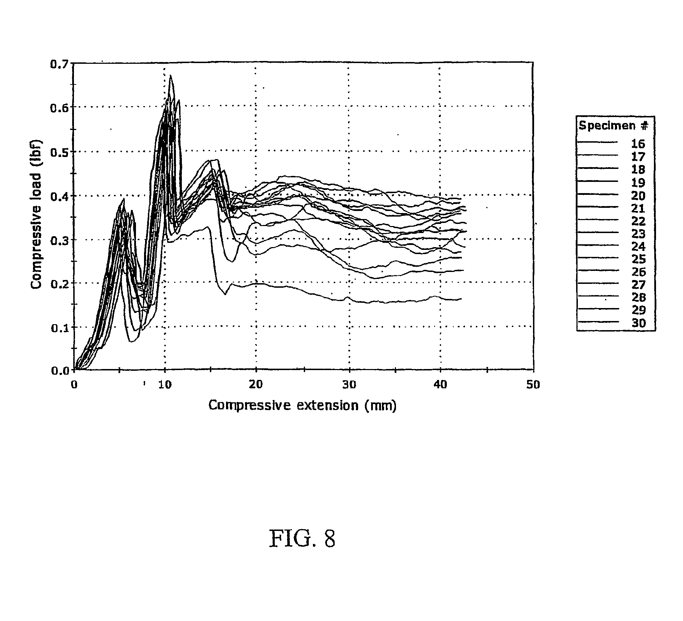

The data of Table 2 is illustrated graphically in Fig. 7 for specimens 1 to 15, and in Fig. 8 for specimens 16 to 30 of the reference apparatus.

As demonstrated by the simulations, considerably more force is required to enter the skin and underlying tissue when utilizing the reference apparatus when compared to the inventive apparatus. This is particularly true at the point where the outer sleeve initially penetrates the skin. This is illustrated by the respective curves shown in Figs. 5-8. The x-axis of the graphs represents the compressive extension, or in other words, the length of insertion (in mm) of the apparatus through the skin. The y-axis of the graph represents the compressive load, or in other words, the force (in N) that is exerted by the physician upon insertion of the apparatus through the skin. As shown in each of the graphs of Figs. 5-8, the compressive load gradually builds as the apparatus is advanced into the skin until the inner cannula has initially penetrated the skin. This is represented by the first peak in each of the figures. In both the reference apparatus and the inventive apparatus (e.g., in each of the Figs. 5-8), the first peak indicates a maximum compressive load in the vicinity of about 1.56 N (0.35 lbf).

Upon insertion of this cannula through the skin an immediate decrease in force is observed once the initial penetration, or puncture, has been completed. As the apparatus is further inserted, the force once again builds, to represent the force required for the outer sleeve to penetrate the skin. As illustrated in Figs. 7 and 8, and as documented in Table 2, a maximum compressive load that varies from 2.36 N to 2.80 N (0.53 to 0.63 lbf) is required in the thirty reference specimens when the outer sleeve penetrates the skin. The mean value of maximum compressive load of all thirty reference specimens is indicated as 2.67 N (0.60 lbf). This is best shown in the figures as the large (second) peak in the vicinity of 2.67 N (0.60 lbf).

For comparison, as illustrated in Figs. 5 and 6, and as documented in Table 1, a much smaller maximum compressive load is required when the outer sleeve penetrates the skin with the inventive apparatus. This graphically shown by viewing the second peak in each of these figures. This peak illustrates that a compressive load of only about 0.89 N to 1.33 N (0.20 to 0.30 lbf) is generally required at the insertion point of the outer sleeve. The reduction in force required with the inventive apparatus when compared to the reference apparatus is dramatically indicated by the absence of the large second peak in the inventive specimens (Figs. 5 and 6). This indicates a much smoother insertion when compared to the reference apparatus. The mean value of the maximum compressive load of all thirty of the inventive specimens is indicated as 1.56 N (0.35 lbf). In most of the inventive specimens, the maximum compressive load does not even occur at this insertion point (second peak), but rather, later during the insertion procedure as the tapered outer sleeve continues to be pushed through the skin.

Appendices 1 and 2 attached hereto provide raw data that corresponds to the curves of Figs. 5-8. Specifically, Appendix 1 includes data from reference Specimens 1-30. This data is graphically illustrated in Fig. 7 (reference specimens 1-15) and Fig. 8 (reference specimens 16-30). Appendix 2 includes data from Specimens 1-30 of the inventive apparatus. This data is graphically illustrated in Fig. 5 (inventive apparatus specimens 1-15) and Fig. 6 (inventive apparatus specimens 16-30). The "load" column in the appendices is specified in units of kgf. The data in the appendices could also be averaged and plotted in that manner. In this event, one averaged curve corresponds to the data for the reference apparatus, and one averaged curve corresponds for readings related to the inventive apparatus. The inventive apparatus comprising the outer sleeve and inner cannula can be provided as a combination, or as separate components. Similarly, all of the components discussed herein can be provided as a kit, or as separate components. The individual components are well known, and, other than the distinctions specified hereinabove, may be formed in conventional manner and of well known compositions. Although the outer sleeve and inner cannula can be formed of any materials suitable for their intended use, preferably they will be formed from a suitable polymer, such as polyethylene.

It is therefore intended that the foregoing detailed description be regarded as illustrative rather than limiting, and that it be understood that it is the following claims, including all equivalents, that are intended to define the scope of this invention.

## Claims

1. A low profile introducer apparatus, comprising:
an outer sleeve having proximal and distal open ends, and having a lumen extending longitudinally therethrough, said outer sleeve having a profile such that at least a portion of said outer sleeve tapers diametrically inwardly toward said outer sleeve distal end at an angle not exceeding about 2.5° relative to the longitudinal axis of the apparatus, said outer sleeve having an outer diameter that does not exceed about 6 French and having a distal open end sized such that a first wire guide is receivable therethrough, said distal open end of said outer sleeve having a wall thickness not exceeding 0.076 mm (0.003 inch); and
an inner cannula having proximal and distal open ends, and having a lumen extending longitudinally therethrough, said inner cannula sized to be receivable within the lumen of said outer sleeve, said inner cannula having a portion that tapers toward the inner cannula distal end, said inner cannula distal open end sized such that a second wire guide is receivable through said inner cannula distal open end, said tapered distal portion of said inner cannula extending distal to the distal open end of said outer sleeve and having a profile such that a generally smooth diametrical transition is provided between said outer sleeve tapered portion said inner cannula open end.

2. The apparatus of claim 1, wherein said outer sleeve tapered portion comprises the distal 5 to 50 mm of said outer sleeve.

3. The apparatus of claim 2, wherein said outer sleeve tapered portion comprises about the distal 15 mm of said outer sleeve.

4. The apparatus of any one of the preceding claims, wherein said outer sleeve tapered portion is tapered at an angle of between 0.5° and 2°.

5. The apparatus of claim 4, wherein said outer sleeve tapered portion is tapered at an angle of about 1°.

6. The apparatus of any one of the preceding claims, wherein said distal open end of said outer sleeve has a wall thickness of between 0.0127 mm (0.0005inch) and 0.0381 mm (0.0015 inch).

7. The apparatus of claim 6, wherein said distal open end of said outer sleeve has a wall thickness of about 0.0254 mm (0.001 inch).

8. The apparatus of any one of the preceding claims, wherein said outer sleeve has an outer diameter between about 1.32 mm and 1.98mm (4 and 6 French).

9. The apparatus of any one of the preceding claims, wherein said outer sleeve has an outer diameter that does not exceed 1.65 mm (5 French).

10. The apparatus of claim 8, wherein said diameter is about 1.65 mm (5 French).

11. An introducer kit comprising:
an introducer needle, said introducer needle having an outer diameter that does not exceed about 0.81mm (21 gauge, 0.032 inch), and an inner diameter that does not exceed about 0.46 mm (0.018 inch);
a first wire guide, said first wire guide having an outer diameter not less than about 0.89 mm, (0.35 inch);
a second wire guide, said second wire guide having an outer diameter not greater than about 0.46 mm (0.018 inch), said second wire guide receivable in a bore of said introducer needle; and low profile introducer apparatus in accordance with any one of the preceding claims.

## Patentansprüche

1. Niederprofil-Einführungsgerät, das Folgendes umfasst: eine Außenhülse mit proximalen und distalen offenen Enden und mit einem Lumen, das sich in Längsrichtung durch es hindurch erstreckt, wobei die Außenhülse ein solches Profil aufweist, das sich zumindest ein Teil der Außenhülse diametral einwärts in einem Winkel von höchstens ungefähr 2,5° relativ zur Längsachse des Geräts zum distalen Ende der Außenhülse hin verjüngt, wobei die Außenhülse einen Außendurchmesser aufweist, der ungefähr 6 French nicht übersteigt und ein distales offenes Ende aufweist, dessen Größe so bemessen ist, dass ein erster Führungsdraht durch es hindurch aufgenommen werden kann, wobei das distale offene Ende der Außenhülse eine Wanddicke von höchstens 0,076 mm (0,003 Zoll) aufweist; und eine Innenkanüle mit proximalen und distalen offenen Enden und mit einem Lumen, das sich in Längsrichtung durch es hindurch erstreckt, wobei die Innenhülse eine solche Größe aufweist, dass sie im Lumen der Außenhülse aufgenommen werden kann, wobei die Innenkanüle einen Abschnitt aufweist, der sich zum distalen Ende der Innenkanüle hin verjüngt, wobei das distale offene Ende der Innenkanüle so bemessen ist, dass ein zweiter Führungsdraht durch das distale offene Ende der Innenkanüle hindurch aufgenommen werden kann, wobei der verjüngte distale Abschnitt der Innenkanüle sich distal zum distalen offenen Ende der Außenhülse erstreckt und ein solches Profil aufweist, dass zwischen dem verjüngten Abschnitt der Außenhülse und dem offenen Ende der Innenkanüle ein allgemein glatter diametraler Übergang entsteht.

2. Gerät nach Anspruch 1, worin der verjüngte Abschnitt der Außenhülse die distalen 5 bis 50 mm der Außenhülse umfasst.

3. Gerät nach Anspruch 2, worin der verjüngte Abschnitt der Außenhülse ungefähr die distalen 15 mm der Außenhülse umfasst.

4. Gerät nach einem der vorhergehenden Ansprüche, worin der verjüngte Abschnitt der Außenhülse in einem Winkel zwischen 0,5° und 2° verjüngt ist.

5. Gerät nach Anspruch 4, worin der verjüngte Abschnitt der Außenhülse in einem Winkel von ungefähr
1° verjüngt ist.

6. Gerät nach einem der vorhergehenden Ansprüche, worin das distale offene Ende der Außenhülse eine Wanddicke zwischen 0,0127 mm (0,0005 Zoll) und 0,0381 mm (0,0015 Zoll) aufweist.

7. Gerät nach Anspruch 6, worin das distale offene Ende der Außenhülse eine Wanddicke von ungefähr 0,0254 mm (0,001 Zoll) aufweist.

8. Gerät nach einem der vorhergehenden Ansprüche, worin die Außenhülse einen Außendurchmesser zwischen ungefähr 1,32 mm und 1,98 mm (4 bis 6 French) aufweist.

9. Gerät nach einem der vorhergehenden Ansprüche, worin die Außenhülse einen Außendurchmesser von höchstens 1,65 mm (5 French) aufweist.

10. Gerät nach Anspruch 8, worin der Durchmesser ungefähr 1,65 mm (5 French) beträgt.

11. Einführungskit, das Folgendes umfasst: eine Einführungsnadel, wobei die Einführungsnadel einen Außendurchmesser von höchstens ungefähr 0,81 mm (21 Gauge, 0,032 Zoll) und einen Innendurchmesser von höchstens ungefähr 0,46 mm (0,018 Zoll) aufweist; einen ersten Führungsdraht, wobei der erste Führungsdraht einen Außendurchmesser von mindestens ungefähr 0,89 mm (0,35 Zoll) aufweist; einen zweiten Führungsdraht, wobei der zweite Führungsdraht einen Außendurchmesser von höchstens ungefähr 0,46 mm (0,018 Zoll) aufweist, wobei der zweite Führungsdraht in einer Bohrung in der Einführungsnadel aufgenommen werden kann; und ein Niederprofil-Einführungsgerät nach einem der vorhergehenden Ansprüche.

## Revendications

1. Appareil introducteur peu encombrant, comportant :
un manchon externe muni d'extrémités proximale et distale ouvertes, et ayant une lumière s'étendant longitudinalement à travers celui-ci, ledit manchon externe ayant un profil tel qu'au moins une portion dudit manchon externe diminue progressivement diamétralement vers l'intérieur vers ladite extrémité distale du manchon externe à une orientation angulaire ne dépassant pas environ 2,5° par rapport à l'axe longitudinal de l'appareil, ledit manchon externe ayant un diamètre externe qui ne dépasse pas environ 6 French et ayant une extrémité distale ouverte dimensionnée de telle sorte qu'un premier fil-guide peut y être reçu, ladite extrémité distale ouverte dudit manchon externe ayant une épaisseur de paroi ne dépassant pas 0,076 mm (0,003 pouce) ; et
une canule interne munie d'extrémités proximale et distale ouvertes, et ayant une lumière s'étendant longitudinalement à travers celle-ci, ladite canule interne étant dimensionnée pour pouvoir être reçue à l'intérieur de la lumière dudit manchon externe, ladite canule interne ayant une portion diminuant progressivement vers l'extrémité distale de la canule interne, ladite extrémité distale de la canule interne étant dimensionnée de telle sorte qu'un deuxième fil-guide peut être reçu dans ladite extrémité distale ouverte de la canule interne, ladite portion distale conique de ladite canule interne s'étendant à distance de l'extrémité distale ouverte dudit manchon externe et ayant un profil tel qu'une transition diamétrale en général lisse est fournie entre ladite portion conique du manchon externe et ladite extrémité ouverte de la canule interne.

2. Appareil selon la revendication 1, dans lequel ladite portion conique du manchon externe comporte la partie distale de 5 à 50 mm dudit manchon externe.

3. Appareil selon la revendication 2, dans lequel ladite portion conique du manchon externe comporte la partie distale d'environ 15 mm dudit manchon externe.

4. Appareil selon l'une quelconque des revendications précédentes, dans lequel ladite portion conique du manchon externe diminue progressivement à une orientation angulaire située entre 0,5 et 2°.

5. Appareil selon la revendication 4, dans lequel ladite portion conique du manchon externe diminue progressivement à une orientation angulaire d'environ 1°.

6. Appareil selon l'une quelconque des revendications précédentes, dans lequel ladite extrémité distale ouverte dudit manchon externe a une épaisseur de paroi se situant entre 0,0127 mm (0,0005 pouce) et 0,0381 mm (0,0015 pouce).

7. Appareil selon la revendication 6, dans lequel ladite extrémité distale ouverte dudit manchon externe a une épaisseur de paroi d'environ 0,0254 mm (0, 001 pouce).

8. Appareil selon l'une quelconque des revendications précédentes, dans lequel ledit manchon externe a un diamètre externe se situant entre environ 1,32 mm et 1,98 mm (4 et 6 French) .

9. Appareil selon l'une quelconque des revendications précédentes, dans lequel ledit manchon externe a un diamètre externe qui ne dépasse pas 1,65 mm (5 French).

10. Appareil selon la revendication 8, dans lequel ledit diamètre est d'environ 1,65 mm (5 French).

11. Ensemble introducteur comportant :
une aiguille d'introducteur, ladite aiguille d'introducteur ayant un diamètre externe qui ne dépasse pas environ 0,81 mm (calibre 21, 0,032 pouce), et un diamètre interne qui ne dépasse pas environ 0,46 mm (0,018 pouce) ;
un premier fil-guide, ledit premier fil-guide ayant un diamètre externe qui n'est pas inférieur à environ 0,89 mm, (0,35 pouce) ;
un deuxième fil-guide, ledit deuxième fil-guide ayant un diamètre externe qui n'est pas supérieur à environ 0,46 mm (0,018 pouce), ledit deuxième fil-guide pouvant être reçu dans un alésage de ladite aiguille d'introducteur ; et un appareil introducteur peu encombrant selon l'une quelconque des revendications précédentes.
